# EUROPEAN PATENT APPLICATION

(11) **EP 3 498 260 A1**
(43) Date of publication of application: **19.06.2019**
(21) Application number: 17206595.5
(22) Date of filing: 12.12.2017
(51) Int. Cl.: A61K 9/00, A61K 31/4704, A61K 9/20

(54) **ORAL GASTRORETENTIVE SUSTAINED-RELEASE PHARMACEUTICAL FORMULATION**

(71) Applicant: Pro.Med.CS Praha A.S., 14000 Praha 4 - Michle (CZ)
(72) Inventor: Lisal, Jiri, 18200 Praha 8 (CZ); Jamaeinová, Jaroslava, 14000 Praha 4 (CZ); Vybiralova, Zuzana, 50001 Hradec Kralove (CZ); Trnkova, Jana, 53352 Stare Hradiste (CZ); Rezac, Jaroslav, 27711 Neratovice (CZ); Svoboda, Zbynek, 50303 Holohlavy, Smirice (CZ)
(74) Representative: Hartvichova, Katerina

(57) **Abstract**

The present invention provides a gastroretentive pharmaceutical composition for oral administration with sustained release comprising at least one active ingredient effective in stomach (preferably rebamipide), which further comprises at least one pharmaceutically acceptable auxiliary substance capable of swelling the pharmaceutical composition to density below 1 g/cm³ at pH between 1.5 and 3.5, at least one pharmaceutically acceptable auxiliary substance capable of ensuring sustained release of the active ingredient, and at least one pharmaceutically acceptable component capable of forming carbon dioxide upon contact with gastric juices.

The pharmaceutical composition can be prepared by wet granulation and/or dry homogenization of the active ingredient with the auxiliary substances and components.

## Description

### Field of Art

The present invention relates to an oral gastroretentive sustained-release pharmaceutical formulation, more particularly to gastroretentive tablets containing rebamipide.

### Background Art

Rebamipide is a gastroprotective drug, chemically described as an amino acid derivative of 2(1H)-quinolinone. This drug is used for gastroprotective action and for efficacy to heal gastric ulcers. This drug stimulates prostaglandin generation in gastric mucosa and improves not only the speed but also the quality of ulcer healing. In addition, it protects the gastric mucosa against acute injury caused by various noxious and ulcerogenic factors. Based on these experimental results, rebamipide had been subsequently tested in several clinical trials and approved in Japan for therapeutic use in patients with gastric ulcers and patients with acute gastritis. Rebamipide improved the quality of gastric ulcer healing and reduced future ulcer recurrence. A number of basic research studies have been performed to clarify the mechanisms of rebamipide's action. These studies demonstrated unique properties of rebamipide and convincingly showed that it increases gastric mucus glycoprotein components, stimulates migration and proliferation of wounded epithelial cell monolayers, increases expression of epidermal growth factor and its receptor in normal and ulcerated gastric mucosa, and scavenges active oxygen radicals. The drug also attenuates the activity of neutrophils and the production of inflammatory cytokines stimulated by NSAIDs and/or H. pylori. Therefore, rebamipide can contribute to the management of patients who are taking NSAIDs or are infected with H. pylori. The inhibition of immunoinflammatory responses by rebamipide in H. pylori-infected patients may prevent development of gastritis, peptic ulcer disease, its recurrence, and possibly gastric cancer. Moreover, rebamipide may enhance eradication of H. pylori-infection using standard eradication therapy.
Conventional dosage forms containing rebamipide developed in the past are usually immediate release formulations of 100 mg of the active substance for oral admistration taken 3 times per day. Main disadvantages of such immediate release forms is administration 3 times daily and also a fact that the active substance should act also locally in stomach musoca which is not possible for a dosage form with a very short residence time in stomach. It is therefore the aim of the present invention to provide a pharmaceutical formulation which would ensure sustained release of the active ingredient, ideally over 16-24 hours, and which would be retained in the stomach over the whole period of release of the active compound.

### Disclosure of the Invention

The present invention relates to formulation of a novel oral sustained-release dosage form containing an active ingredient effective in stomach, preferably containing rebamipide as the active ingredient (active pharmaceutical ingredient, API). The developed formulation enables to prolong the drug residence time in stomach where the active pharmaceutical ingredient is slowly released from the dosage form and a part of it locally acts on the mucosa. The retention of the pharmaceutical form in stomach prevents its moving further through the gastrointestinal tract (GIT) and the undesired release of the active ingredient in other parts of the GIT than stomach. There are three mechanisms enabling retentive and sustained release behaviour of the dosage form involved in this invention. First mechanism is based on carbon dioxide which is generated when the dosage form comes in contact with acidic gastric fluids. This process results in floating of the dosage form. Second mechanism is swelling of dosage form to density below 1 g/cm³ at pH between 1.5 and 3.5 which is enabled by functional polymers. Third mechanism is mucoadhesion of the dosage form containing alginate-based polymer.
Both *in-vitro* and *in-vivo* tests prove the desired characteristics of the dosage form such as gastroretentive behaviour, sustained release of the API enabling 24 h continuous release, and mucoadhesion of the dosage form or part of it and thus locally action on stomach mucosa. Another important benefit of this invention is replacing several dosage units (e.g., for rebamipide 3 dosage units) of immediate release form by 1 dosage unit of sustained release form.

The present invention provides a gastroretentive pharmaceutical composition for oral administration with sustained release comprising at least one active ingredient effective in stomach, at least one pharmaceutically acceptable auxiliary substance capable of swelling the pharmaceutical composition to density below 1 g/cm³ at pH between 1.5 and 3.5, at least one pharmaceutically acceptable auxiliary substance capable of ensuring sustained release of the active ingredient, and at least one pharmaceutically acceptable component capable of forming carbon dioxide upon contact with gastric juices.

The composition is in a form suitable for oral administration, such as tablet, capsule, or granule.

The "active ingredient effective in stomach" means a prophylactically or therapeutically active substance which needs to be or shall be ingested or absorbed through stomach mucosa in order to be effective, or which acts prophylactically or therapeutically directly in stomach, e.g. by acting on constituents of the stomach mucosa or on components of the content of the stomach.

In one preferred embodiment, the active ingredient effective in stomach is rebamipide.

The pharmaceutically acceptable auxiliary substance capable of swelling the pharmaceutical composition to density below 1 g/cm³ at pH between 1.5 and 3.5 ensures that the pharmaceutical composition remains in stomach. Such swelled pharmaceutical composition floats on the surface of the stomach content due to its density below 1 g/cm³, being thereby retained in the stomach and not moving further into small intestine. The swelling must take place in the stomach, and therefore it is important that it takes place at pH between 1.5 and 3.5 - that is, in the acidic conditions of gastric juices.

The auxiliary substance capable of swelling the pharmaceutical composition to the required density is preferably crosslinked polyvinyl N-pyrrolidone (crospovidone). Preferably, crospovidone is present in the amount of 5 to 15 wt. %, relative to the total weight of the composition.

The auxiliary substance capable of ensuring sustained release of the active ingredient is a component which typically forms a matrix from which the active ingredient is released in a controlled manner, or a component which encapsulates the active ingredient in order to slow down its release. Such auxiliary substances ensuring sustained release may include cellulose and cellulose ethers such as hydroxypropyl cellulose, hydroxypropyl methylcellulose, sodium carboxymethylcellulose, ethylcellulose; alginic acid and alginates, such as propylene glycol alginate or sodium alginate; polymethacrylate polymers (eudragit); guar gum; xanthan gum.

Preferably, the auxiliary substances capable of ensuring sustained release of the active ingredient are selected from cellulose and cellulose ethers, alginic acid and alginates, and combinations thereof.

Most preferably, the auxiliary substances capable of ensuring sustained release of the active ingredient are a combination of a cellulose ether such as hydroxypropylcellulose, hydroxypropyl methylcellulose, sodium carboxymethylcellulose, ethylcellulose, with an alginate such as propylene glycol alginate.

The amount of the alginate is preferably between 10 and 50 wt. %, more preferably between 15 and 35 wt. %, relative to the total weight of the composition.

The amount of the cellulose ether is preferably between 3 and 15 wt. %, more preferably between 5 and 10 wt. %, relative to the total weight of the composition.

The pharmaceutically acceptable component capable of forming carbon dioxide upon contact with gastric juices may be selected from carbonates and hydrogen carbonates of alkali metals and alkaline earth metals, e.g., sodium hydrogencarbonate, sodium carbonate, potassium hydrogencarbonate, potassium carbonate, calcium carbonate, calcium hydrogencarbonate. The production of carbon dioxide ensures that the pharmaceutical form is quickly propelled towards surface and it may also increase the speed of swelling, thereby reducing the risk that the pharmaceutical form is carried further into small intestine together with the stomach content before achieving a sufficiently swelled-up form. The amount of the carbonates and/or hydrogen carbonates of alkali metals and/or alkaline earth metals is preferably in the range from 2 to 15 wt. %, more preferably from 5 to 10 wt. %, relative to the total weight of the composition.

Yet further, it is advantageous when the pharmaceutical composition comprises at least one pharmaceutically acceptable component capable of adhering to the stomach mucose. The presence of a mucoadhesive component further improves the retainability of the pharmaceutical composition in the stomach. Furthermore, it is especially suitable for active ingredients that need to be adsorbed by stomach mucosa. Suitable mucoadhesive components include alginates such as propylene glycol alginate.

The pharmaceutical composition may further contain other commonly used auxiliary substances, such as lubricants (e.g., magnesium stearate, calcium stearate, stearic acid, sodium laurylsulphate), binders, fillers/diluents (e.g., saccharide alcohols such as mannitol, sorbitol, xylitol; lactose, starch, cellulose), disintegrants (e.g., sodium cross-carmelose, sodium carboxymethyl starch), coatings.

The amount of the active ingredient effective in stomach is preferably in the range of 20 to 50 wt. %, more preferably 30 to 40 wt. %, relative to the total weight of the composition.

In one embodiment, a suitable pharmaceutical composition comprises at least one active ingredient effective in stomach, and the following pharmaceutically acceptable auxiliary substances and components:
- at least one cellulose ether such as hydroxypropylcellulose, hydroxypropyl methylcellulose, ethylcellulose;
- at least one alginate such as propylene glycol alginate, sodium alginate, or alginic acid;
- at least one carbon dioxide-forming component such as sodium hydrogencarbonate, sodium carbonate, calcium hydrogencarbonate, calcium carbonate,
- crospovidone,
- at least one lubricant such as magnesium stearate, calcium stearate, stearic acid, sodium laurylsulphate,
- at least one filler/diluent and/or disintegrant such as saccharide alcohols (mannitol, sorbitol, xylitol), lactose, starch, cellulose, sodium cross-carmelose, sodium carboxymethyl starch.

In a more preferred embodiment, a suitable pharmaceutical composition is in the form of a tablet, and comprises at least one active ingredient effective in stomach, preferably rebamipide, in an amount of 20-50 wt. %, more preferably 30-40 wt.%, and the following pharmaceutically acceptable auxiliary substances and components:
- 3-15 wt.% of at least one cellulose ether such as hydroxypropyl methylcellulose, hydroxypropyl cellulose, ethylcellulose,
- 10-50 wt.% of at least one alginate such as propylene glycol alginate, sodium alginate, or alginic acid,
- 2-15 wt.% of at least one carbon dioxide-forming component such as sodium hydrogencarbonate, sodium carbonate, calcium hydrogencarbonate, calcium carbonate, - 5-15 wt.% of crospovidone,
- 0.5-3 wt.% of at least one lubricant such as magnesium stearate, calcium stearate, stearic acid, sodium laurylsulphate,
- 5-20 wt.% of at least one filler/diluent and/or disintegrant such as saccharide alcohols (mannitol, sorbitol, xylitol), lactose, starch, cellulose, sodium cross-carmelose, sodium carboxymethyl starch.

In one particular embodiment, a suitable pharmaceutical composition is in the form of a tablet, and comprises at least one active ingredient effective in stomach, preferably rebamipide, in an amount of 30-40 wt.% and the following pharmaceutically acceptable auxiliary substances and components:
- 3-15 wt.% of hydroxypropyl methylcellulose and/or hydroxypropyl cellulose,
- 10-50 wt.% of propylene glycol alginate, sodium alginate, and/or alginic acid,
- 5-15 wt.% of sodium hydrogencarbonate, sodium carbonate, and/or calcium carbonate,
- 5-15 wt.% of crospovidone,
- 0.5-3 wt.% of magnesium stearate, calcium stearate, and/or stearic acid,
- 5-20 wt.% of mannitol, sorbitol, xylitol, lactose, starch, and/or sodium carboxymethyl starch.

In one specific embodiment, a suitable pharmaceutical composition is in the form of a tablet, and comprises at least one active ingredient effective in stomach, preferably rebamipid, in an amount of 30-40 wt.% and the following pharmaceutically acceptable auxiliary substances and components:
- 3-15 wt.% of hydroxypropyl methylcellulose and/or hydroxypropyl cellulose,
- 10-50 wt.% of propylene glycol alginate,
- 5-15 wt.% of sodium hydrogencarbonate, sodium carbonate, and/or calcium carbonate,
- 5-15 wt.% of crospovidone,
- 0.5-3 wt.% of magnesium stearate, calcium stearate, and/or stearic acid,
- 5-20 wt.% of mannitol, sorbitol, and/or xylitol.

In one specific embodiment, a suitable pharmaceutical composition is in the form of a tablet, and comprises 30-40 wt.% of rebamipide and the following pharmaceutically acceptable auxiliary substances and components:
- 5-10 wt.% of hydroxypropyl methylcellulose,
- 15-35 wt.% of propylene glycol alginate,
- 5-10 wt.% of sodium hydrogencarbonate, sodium carbonate, and/or calcium carbonate,
- 5-15 wt.% of crospovidone,
- 0.5-3 wt.% of magnesium stearate, calcium stearate, and/or stearic acid,
- 5-20 wt.% of mannitol, sorbitol, and/or xylitol.

The pharmaceutical compositon of the present invention may be prepared by wet granulation and/or dry homogenization of the active ingredient with the auxiliary substances and components.

In one preferred embodiment, the pharmaceutical composition is prepared by wet granulation of the active ingredient, preferably rebamipide, with filler/diluent and crospovidone, drying, and homogenization of the thus obtained granules with cellulose ether, alginate, crospovidone, carbon dioxide-forming component and lubricant. The homogenization is preferably carried out in three steps, in this order: homogenization of the granulate with cellulose ether, homogenization of the thus obtained homogenizate with alginate and carbon-dioxide-forming component, and homogenization of the thus obtained homogenizate with the lubricant.

The present invention thus provides a reliable gastroretentive sustained-release formulation which is inexpensive and does not require complex production methods.

### Brief description of drawings

Figure 1 shows the mucoadhesivity of the tablet of Example 1 to gastric mucosa of a pig.
Figure 2 shows the dissolution profile of the tablet of Example 1 (USP 2 method)
Figure 3 shows the dissolution of the tablet of Example 1 during simulated gastric environment test (% cumulative dissolved, USP 3 method
Figure 4 shows the small particles of the tablet of Example 1 during simulated gastric environment test (% cumulative passed through the screen 0.84 mm undissolved, USP 3 method).
Figure 5 shows part of the tablet of Example 1 available for systemic circulation (USP 3 method).
Figure 6 shows the dissolution profile of the tablet of Example 2 (USP 2 method).
Figure 7 shows the dissolution of the tablet of Example 2 during simulated gastric environment test (% cumulative dissolved, USP 3 method).
Figure 8 shows the small particles of the tablet of Example 2 during simulated gastric environment test (% cumulative passed through the screen 0.84 mm undissolved. USP 3 method).
Figure 9 shows part of the tablet of Example 2 available for systemic circulation (USP 3 method).
Figure 10 shows the dissolution profile of the tablet of Example 3 (USP 2 method).
Figure 11 shows the dissolution profile of the tablet of Example 4 (USP 2 method).
Figure 12 shows the dissolution profile of the tablet of Example 5 (USP 2 method).
Figure 13 shows the dissolution profile of the tablet of Example 6 (USP 2 method).
Figure 14 shows the dissolution profile of the tablet of Example 7 (USP 2 method).

### Examples of carrying out the invention

### Example 1

**Tablet composition 10-110816**

| **10-110816** | amount in 1 tablet [mg] | amount in 1 tablet [%] | function |
|---|---|---|---|
| Rebamipide | 300.00 | 32.43 | API |
| Hydroxypropyl methylcelulose | 60.00 | 6.49 | Sustained-release agent |
| Mannitol | 100.00 | 10.81 | Filler |
| Crospovidone | 92.00 | 9.95 | Swelling agent |
| Sodium bicarbonate | 60.00 | 6.49 | Gas forming agent |
| Magnesii stearas | 10.00 | 1.08 | Lubricant |
| **Alginate propylene glycol** | **303.00** | **32.76** | Sustained-release agent Mucoadhesive agent |
| **Tablet weight** | 925.00 | 100.00 | |

### Manufacturing procedure:

Rebamipide, Mannitol and Crospovidone were granulated with purified water, after drying the granulate was sieved and homogenized with excipients in 3 steps:
1: Hydroxypropylmethylcelulose is added
2: Alginate propylene glycol and sodium bicarbonate is added
3: Magnesii stearas is added
After homogenization the mixture is compressed/tabletted.

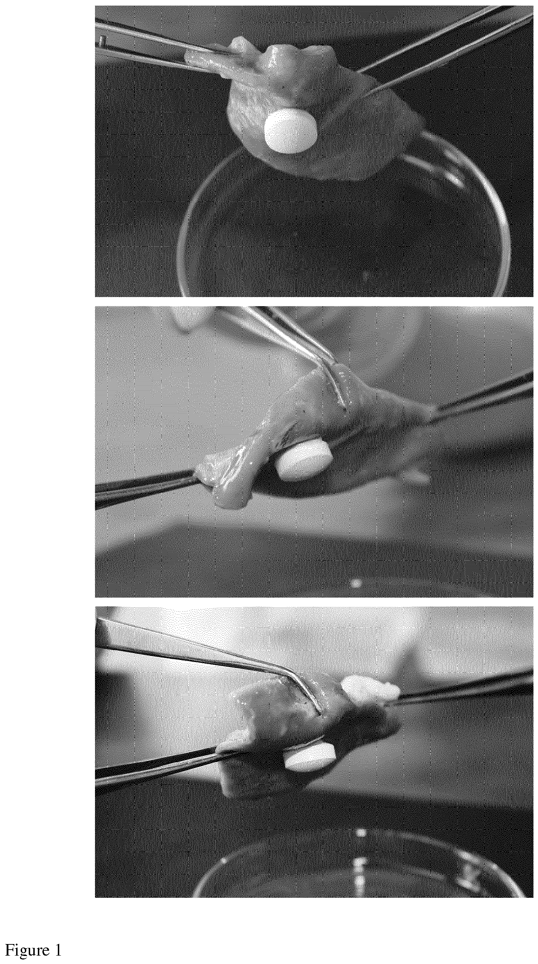

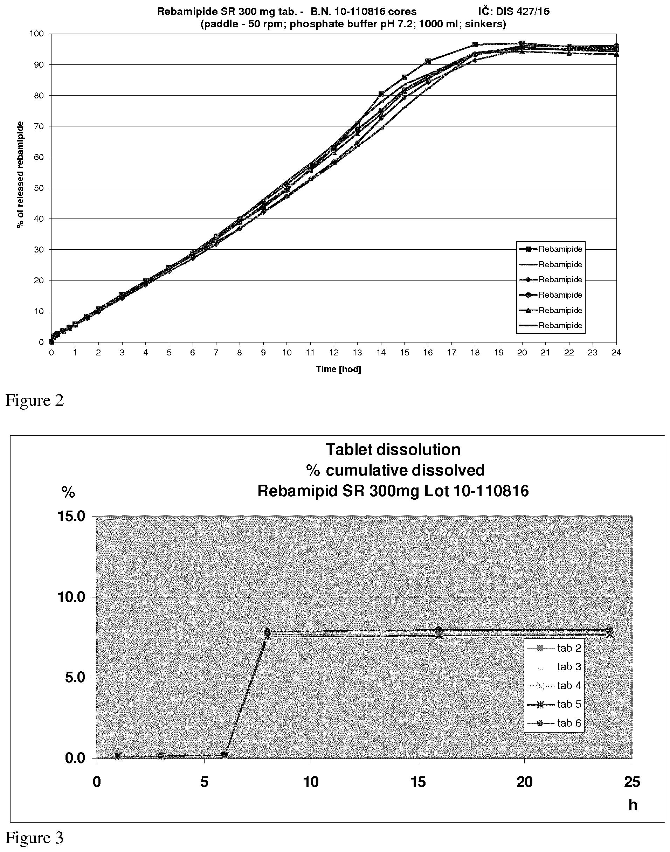

Figure 1 shows the excellent mucoadhesivity of the tablet to gastric mucosa of a pig. Figure 2 shows the dissolution profile of the tablet in phosphate buffer.

### Dissolution tests description

### Dissolution test in USP 2 apparatus (paddle method with sinkers), pH 7.2

A few moments after the tablets were placed in the sinkers, the bubbles formed around the tablets. At 5 minutes, tablets were slightly swollen. At 15 minutes, the tablets were swollen even more, and around the sinkers were small bubbles. In the 120th minute a few small particles were dripping. After 5 hours the tablets were swollen across the spiral, smooth on the surface, occasional clogging of pumps occurred. After 20 hours, the sinkers were empty (in Table No. 2, a piece of tablet remained approximately 21.5 hours). The dissolving medium was stained with loose particles, small particles were at the bottom of the vessels and in the bulge.

At the end of the dissolution, piles of particles were in the bottom of the vessel, at the elevation and at the dis level. The media were small particles.

### Dissolution test in USP 3 apparatus (reciprocating cylinder), pH range 1.3 - 5.5

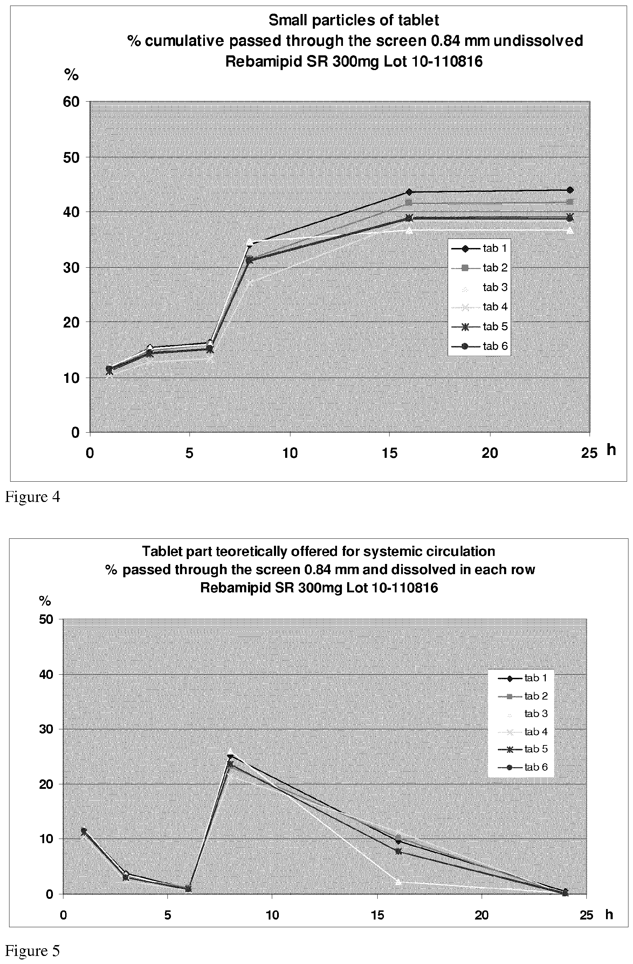

Dissolution test was performed by BIO DIS III method in simulated gastric environment for 24 hours. The test conditions (Table 1) were chosen to approximate the pH range and motility of the human stomach during the day when receiving food and sleep. The results are shown in Tables 2 to 4, and Figures 3 to 5. It was observed that the tablet remains for the whole time of 24 hours on the surface (52.5 ± 2.6 %).

**Table 1: Test conditions**

| **Row** | **Run time, h** | **Dissolution medium** | **pH** | **Dip/min** | **Time interval in media, h** | **Simulated conditions in stomach** |
|---|---|---|---|---|---|---|
| **1** | **1** | phosphate buffer | **3.5** | **30** | **1** | Tablet SR+Smaller food-polysaccharides. HCl secretion, 15 minutes without moving, mixing food, fast moves |
| **2** | **3** | HCL | **1.6** | **15** | **2** | Digestion chyme shift, the gradual neutralization of gastric juice |
| **3** | **6** | HCL | **1.8** | **10** | **3** | Gradual increase in pH in the stomach, chyme shift, slower movements |
| **4** | **8** | phosphate buffer | **5.5** | **30** | **2** | Larger meal with proteins, Hcl secretion, 30 min without movement food mixing fast movements, |
| **5** | **16** | HCL | **1.3** | **15** | **8** | Digestion chyme shift, the gradual neutralization of gastric juice |
| **6** | **24** | HCL | **1.8** | **5** | **8** | Sleep, gradual neutralization of gastric juices, resting movements with low intensity |

**Table 2: Dissolved part of the tablet (tab = tablet), cumulative%**

| **time, h** | **tab 1** | **tab 2** | **tab 3** | **tab 4** | **tab 5** | **tab 6** |
|---|---|---|---|---|---|---|
| **1** | **0.106** | **0.108** | **0.110** | **0.102** | **0.109** | **0.107** |
| **3** | **0.145** | **0.149** | **0.152** | **0.143** | **0.149** | **0.148** |
| **6** | **0.169** | **0.174** | **0.176** | **0.167** | **0.174** | **0.170** |
| **8** | **7.532** | **7.674** | **7.470** | **7.687** | **7.522** | **7.867** |
| **16** | **7.61** | **7.76** | **7.54** | **7.78** | **7.61** | **7.96** |
| **24** | **7.64** | **7.78** | **7.57** | **7.80** | **7.64** | **7.99** |

**Table 3: Insoluble particles <0.84 mm that have fallen through the grid, cumulative%**

| **time, h** | **tab 1** | **tab 2** | **tab 3** | **tab 4** | **tab 5** | **tab 6** |
|---|---|---|---|---|---|---|
| **1** | **11.6** | **11.5** | **11.7** | **10.3** | **11.1** | **11.4** |
| **3** | **15.3** | **14.7** | **15.1** | **12.6** | **14.1** | **14.3** |
| **6** | **16.2** | **15.8** | **15.9** | **13.3** | **15.0** | **15.2** |
| **8** | **34.0** | **31.5** | **34.6** | **27.1** | **31.3** | **31.0** |
| **16** | **43.6** | **41.6** | **36.6** | **38.4** | **38.8** | **38.7** |
| **24** | **43.9** | **41.6** | **36.7** | **38.4** | **39.0** | **38.7** |

**Table 4: Dissolved part of the tablet + undissolved particle <0.84 mm that fell through the grid,% tablet in each row.**

| **time, h** | **tab 1** | **tab 2** | **tab 3** | **tab 4** | **tab 5** | **tab 6** |
|---|---|---|---|---|---|---|
| **1** | **11.67** | **11.57** | **11.81** | **10.45** | **11.22** | **11.52** |
| **3** | **3.75** | **3.29** | **3.50** | **2.32** | **3.04** | **2.97** |
| **6** | **0.99** | **1.13** | **0.79** | **0.67** | **0.89** | **0.83** |
| **8** | **25.15** | **23.17** | **25.93** | **21.37** | **23.65** | **23.55** |
| **16** | **9.62** | **10.19** | **2.14** | **11.34** | **7.63** | **7.76** |
| **24** | **0.40** | **0.03** | **0.10** | **0.05** | **0.20** | **0.08** |

### Example 2

**Tablet composition 11-230916/1**

| **11-230916/1** | amount in 1 tablet [mg] | amount in 1 tablet [%] | function |
|---|---|---|---|
| Rebamipide | 300.00 | 36.14 | API |
| Hydroxypropylmethylcelulose | 60.00 | 7.23 | Sustained-release agent |
| Mannitol | 100.00 | 12.05 | Filler |
| Crospovidone | 92.00 | 11.10 | Swelling agent |
| Sodium bicarbonate | 60.00 | 7.23 | Gas forming agent |
| Magnesii stearas | 16.00 | 1.93 | Lubricant |
| **Alginate propylene glycol** | **202.00** | **24.32** | Sustained-release agent Mucoadhesive agent |
| **Tablet weight** | 830.00 | 100.00 | |

The manufacturing procedure was the same as in Example 1. The mixture was flowable, did not stick.

### Dissolution test in USP 2 apparatus (paddle method with sinkers), pH 7.2

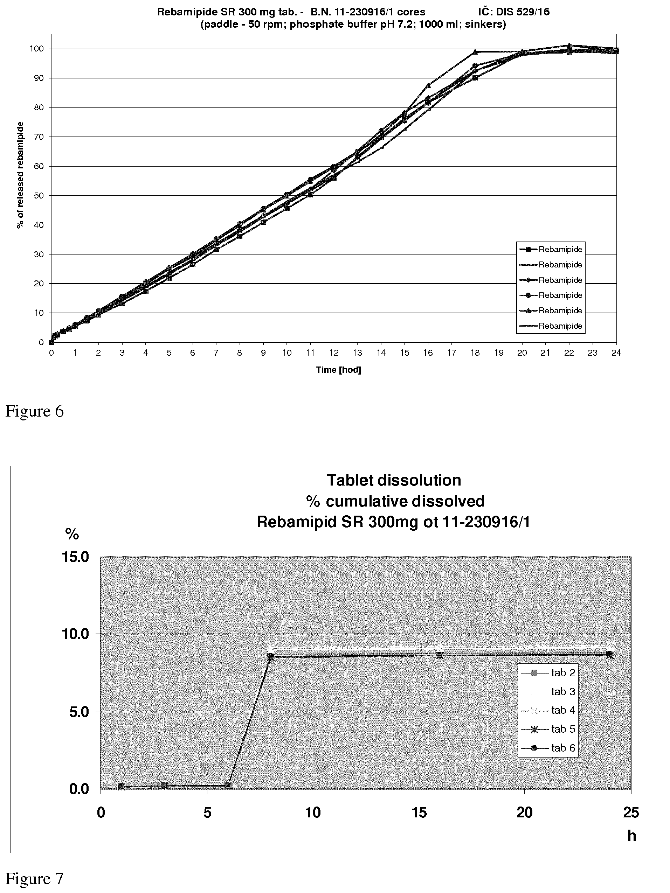

The tablets, after being introduced into pH 7.2, immediately raised, swelled and floated on the surface. Figure 6 shows the dissolution profile of the tablet.

### Dissolution test in USP 3 apparatus (reciprocating cylinder), pH range 1.3 - 5.5

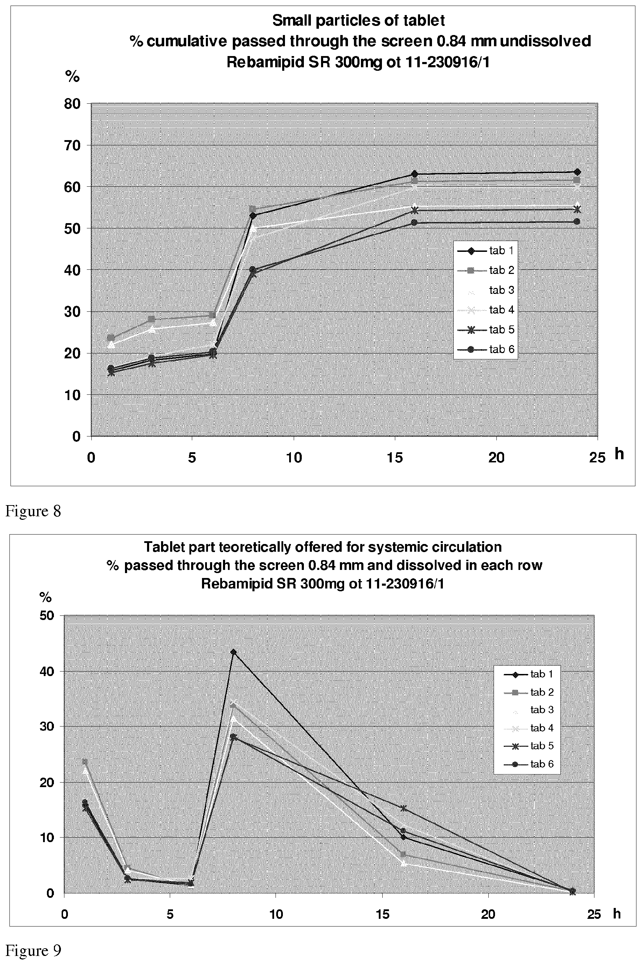

Dissolution test for the tablets of Example 2 was performed by BIO DIS III method in simulated gastric environment for 24 hours under the same conditions as in Example 1. The results are shown in Tables 5 to 7, and in Figures 7 to 9. It was observed that the tablet remains for 24 hours on the surface (31.1 ± 5.1 %).

**Table 5: Dissolved part of tablet, cumulative%**

| **time, h** | **tab 1** | **tab 2** | **tab 3** | **tab 4** | **tab 5** | **tab 6** |
|---|---|---|---|---|---|---|
| **1** | **0.128** | **0.158** | **0.159** | **0.127** | **0.130** | **0.128** |
| **3** | **0.168** | **0.213** | **0.207** | **0.173** | **0.170** | **0.173** |
| **6** | **0.196** | **0.239** | **0.235** | **0.226** | **0.209** | **0.205** |
| **8** | **10.369** | **8.699** | **9.015** | **9.129** | **8.515** | **8.563** |
| **16** | **10.45** | **8.77** | **9.09** | **9.20** | **8.59** | **8.64** |
| **24** | **10.48** | **8.79** | **9.11** | **9.23** | **8.62** | **8.67** |

**Table 6: Unsoluble particles <0.84 mm that have fallen through the grid, cumulative%**

| **time, h** | **tab 1** | **tab 2** | **tab 3** | **tab 4** | **tab 5** | **tab 6** |
|---|---|---|---|---|---|---|
| **1** | **15.8** | **23.4** | **21.9** | **16.6** | **15.2** | **16.3** |
| **3** | **18.3** | **28.0** | **25.9** | **19.2** | **17.6** | **18.9** |
| **6** | **19.7** | **29.1** | **27.3** | **21.9** | **19.4** | **20.3** |
| **8** | **53.0** | **54.5** | **49.9** | **47.5** | **39.1** | **40.1** |
| **16** | **63.0** | **61.3** | **55.2** | **59.7** | **54.3** | **51.2** |
| **24** | **63.4** | **61.5** | **55.4** | **59.9** | **54.4** | **51.5** |

**Table 7: Dissolved part of the tablet + undissolved particle <0.84 mm that fell through the grid,% tablet in each row.**

| **time, h** | **tab 1** | **tab 2** | **tab 3** | **tab 4** | **tab 5** | **tab 6** |
|---|---|---|---|---|---|---|
| **1** | **15.88** | **23.58** | **22.04** | **16.70** | **15.29** | **16.40** |
| **3** | **2.58** | **4.64** | **4.02** | **2.70** | **2.44** | **2.62** |
| **6** | **1.42** | **1.10** | **1.46** | **2.73** | **1.88** | **1.50** |
| **8** | **43.44** | **33.87** | **31.41** | **34.49** | **27.97** | **28.13** |
| **16** | **10.12** | **6.85** | **5.35** | **12.25** | **15.32** | **11.21** |
| **24** | **0.46** | **0.26** | **0.22** | **0.22** | **0.16** | **0.30** |

The dissolution method at pH 7.2 using paddle method (apparatus USP2) was chosen to test the retarding function of the preparation formulation. pH 7.2 represents the sink condition, when the active ingredient is immediately dissolved. Then the dissolution proces is driven by tablet disintegration. Typical slow release dissolution profile of formulation examples 1, 2 is shown in Figures 2 and 6. Rebamipide is slowly released from tablets during 24 hours, the variability is very low.
The dissolution behavior of tablets at lower pH with periodical changes and mechanical stress was tested in USP 3 apparatus (reciprocating cylinder). The stomach pH, when the food is not ingested, is relatively stable, with values lying in the range 1 to 3. At pH 1.3 to 2 rebamipid is practically insoluble, the dissolved part is as low as 0.1%. The tablets are slowly eroded and insoluble particles are released by 1 to 2 % per hour. The fraction of small undissolved particles was expected to pass to the intestine, where at higher pH could be dissolved and available for systemic circulation.
After food intake, a change towards higher pH values up to 5.5 could be expected regarding stomach physiology. During the pH changes at 3.5 - 5.5, the tablet desintegration is enhanced by 11 to 12 % per hour (Example 1) or 16 to 18% per hour (Example 2), and the dissolution reaches up to 10 %. At this pH the dissolved part of rebamipide is available to stomach mucosa, while the eroded particles could pass to the intestine and in higher pH dissolved and transported to systemic circulation.
During 24 hour test, which mimic the periodic pH change and mechanical stress conditions of the stomach, continual release of rebamipide in the form of the undissolved particles and dissolved fraction were observed.

### Example 3 (comparative example)

**Tablet composition 11-230916/2**

| **11-230916/2** | 1tbl. theory in mg | % | function |
|---|---|---|---|
| Rebamipide | 300.00 | 41.20 | API |
| Hydroxypropylmethylcelulose | 60.00 | 8.24 | Sustained-release agent |
| Mannitol | 100.00 | 13.75 | Filler |
| Crospovidone | 92.00 | 12.64 | Swelling agent (Disintegrant) |
| Sodium bicarbonate | 6.00 | 8.24 | Gas forming agent |
| Magnesii stearas | 15.00 | 2.06 | Lubricant |
| **Alginate propylene glycol** | **101.00** | **13.87** | Sustained-release agent Mucoadhesive agent |
| **Tablet weight** | 728.00 | 100 | |

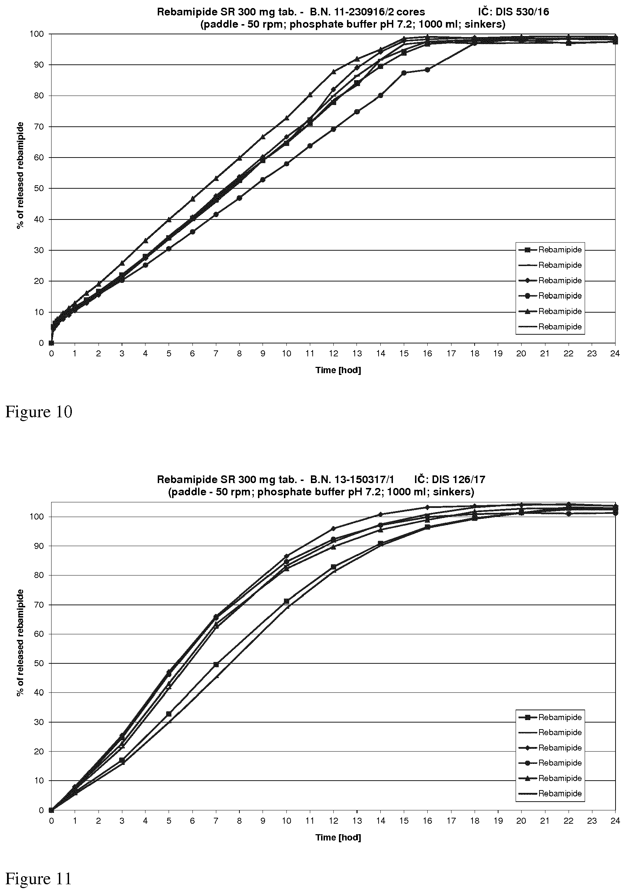

The manufacturing procedure was the same as in Example 1. Lower amount of alginate propylene glycol was used compared to Example 1 and 2. Figure 10 shows the dissolution profile of the tablet.
Dissolution test (USP 2 method) was performed as in examples 1 and 2. After introduction of the tablets into the dissolving vessels, the tablets became swollen and formed spheres. Fine particles were gradually released, which at the end of dissolution were in the bulk, and a small amount of the particles remained at the bottom of the vessels. Dissolution rate was too fast and variability of tablets was worse compared to Example 1 and 2. Therefore dissolution test (USP 3) was not performed.

### Example 4 (comparative example)

**Tablet composition 13-150317/1**

| **13-150317/1** | 1tbl. theory in mg | % | function |
|---|---|---|---|
| Rebamipide | 300.00 | 38.96 | API |
| Hydroxypropylmethylcelulose | 60.00 | 7.79 | Sustained-release agent |
| Mannitol | 100.00 | 12.99 | Filler |
| Crospovidone | 92.00 | 11.95 | Disintegrant |
| **Sodium bicarbonate** | ------ | ------ | Gas forming agent |
| Magnesii stearas | 16.00 | 2.08 | Lubricant |
| Alginate propylene glycol | 202.00 | 26.23 | Sustained-release agent Mucoadhesive agent |
| **Tablet weight** | 770.00 | 100 | |

The manufacturing procedure was the same as in Example 1. The composition did not contain sodium bicarbonate. Figure 11 shows the dissolution profile (USP 2 method) of the tablet.
Dissolution test was performed as in examples 1 and 2. After the tablets were introduced into the dissolution vessels, they became swollen. During the dissolution, very fine particles were released slowly. At the end of the dissolution, the particles were at the bottom of the vessels. Even though the tablets swell and disintegrate, they do not get propelled to the surface just after introduction into the gastric model due to the absence of the gas forming component which results in their remaining at the bottom of the model vessel, in an organism they would move from stomach further to small intestine.
Dissolution rate was too fast and variability of tablets was worse compared to example 1 and 2. Therefore dissolution test (USP 3) was not performed.

### Example 5 (comparative example)

**Tablet composition 13-150317/2**

| **13-150317/2** | 1tbl. theory in mg | % | function |
|---|---|---|---|
| Rebamipide | 300.00 | 33.54 | API |
| Hydroxypropylmethylcelulose | 60.00 | 6.71 | Sustained-release agent |
| Mannitol | 100.00 | 11.18 | Filler |
| Crospovidone | 92.00 | 10.28 | Disintegrant |
| **Sodium bicarbonate** | **124.50** | **13.92** | Gas forming agent |
| Magnesii stearas | 16.00 | 1.79 | Lubricant |
| Alginate propylene glycol | 202.00 | 22.58 | Sustained-release agent Mucoadhesive agent |
| **Tablet weight** | 894.50 | 100 | |

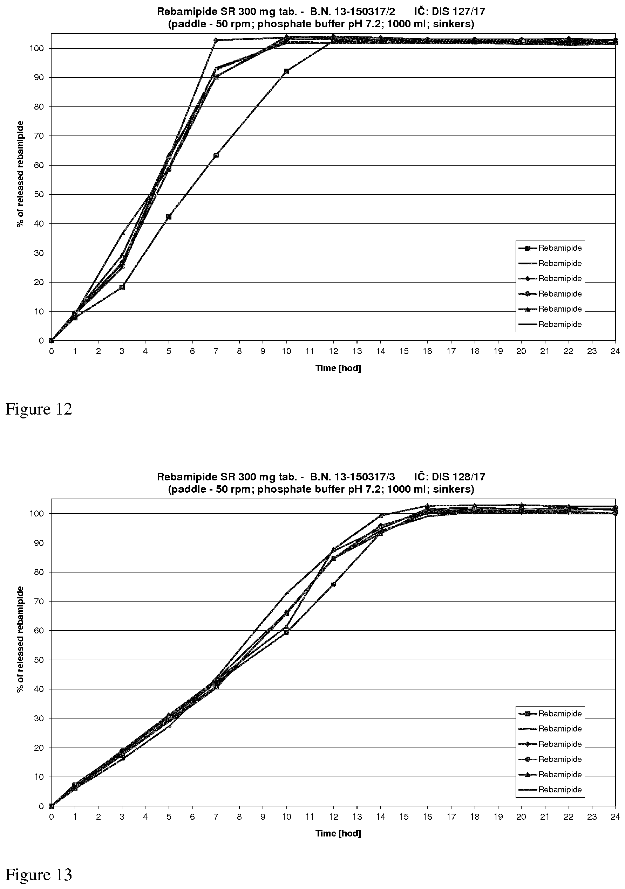

The manufacturing procedure was the same as in Example 1. The composition contained a higher amount of sodium bicarbonate. Figure 12 shows the dissolution profile of the tablet. Dissolution test was performed as in examples 1 and 2. After being introduced into the dissolution vessels, the tablets began to swell very slowly. During the dissolution, the coat was covered in spirals. At the end of the dissolution, a fine particle sludge was present, the fine particles flowing. A very high amount of gas forming agent partially hinders the sustained release.
Dissolution rate was significantly faster and variability of tablets was worse compared to Examples 1 and 2. Therefore, dissolution test (USP 3) was not performed.

### Example 6 (comparative example)

**Tablet composition 13-150317/3**

| **13-150317/3** | 1tbl. theory in mg | % | function |
|---|---|---|---|
| Rebamipide | 300.00 | 37.74 | API |
| **Hydroxypropylmethylcelulose** | **24.90** | **3.13** | Sustained-release agent |
| Mannitol | 100.00 | 12.58 | Filler |
| Crospovidone | 92.00 | 11.57 | Disintegrant |
| Sodium bicarbonate | 60.00 | 7.55 | Gas forming agent |
| Magnesii stearas | 16.00 | 2.01 | Lubricant |
| Alginate propylene glycol | 202.00 | 25.42 | Sustained-release agent Mucoadhesive agent |
| **Tablet weight** | 794.90 | 100 | |

The manufacturing procedure was the same as in Example 1. The composition had a significantly lower content of hydroxypropyl methylcelulose. Figure 13 shows the dissolution profile of the tablet.
Dissolution test was performed as in Examples 1 and 2. After being introduced into the dissolution vessels, the tablets began to swell slowly to form a sphere, from which the fine particles were released, and they started to rise. After 7 hours, the spheres were even enlarged. Through the spirals, even larger particles were floating.
Dissolution rate was too fast compared to Examples 1 and 2. Therefore dissolution test (USP 3) was not performed.

### Example 7 (comparative example)

**Tablet composition 13-150317/4**

| **13-150317/4** | 1tbl. theory in mg | % | function |
|---|---|---|---|
| Rebamipide | 300.00 | 33.54 | API |
| **Hydroxypropylmethylcelulose** | **124.50** | **13.92** | Sustained-release agent |
| Mannitol | 100.00 | 11.18 | Filler |
| Crospovidone | 92.00 | 10.28 | Disintegrant |
| Sodium bicarbonate | 60.00 | 6.71 | Gas forming agent |
| Magnesii stearas | 16.00 | 1.79 | Lubricant |
| Alginate propylene glycol | 202.00 | 22.58 | Sustained-release agent Mucoadhesive agent |
| **Tablet weight** | 894.50 | 100 | |

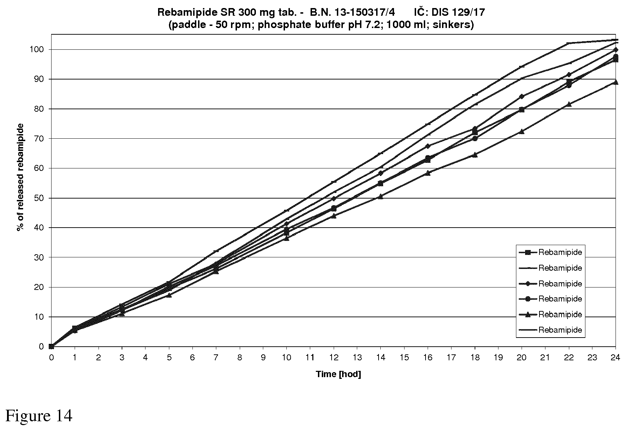

The manufacturing procedure was the same as in Example 1. The composition had a significantly higher content of hydroxypropyl methylcelulose. Figure 14 shows the dissolution profile of the tablet.
Dissolution test was performed as in examples 1 and 2. After being introduced into the dissolution vessels, the tablets swelled to form beads filled with spirals. After 5 hours, the balls were still spiraling. Their surface was coarse-grained and the individual tiny particles released. After 22 hours, several larger particles remained in one vessel, while in the other vessels the number was reduced to half. At the end of dissolution, the tablet residues were as large as the spirals.
Dissolution rate was too slow compared to Examples 1 and 2. Therefore dissolution test (USP 3) was not performed.

## Claims

1. A gastroretentive pharmaceutical composition for oral administration with sustained release comprising at least one active ingredient effective in stomach, **characterized in that** it further comprises at least one pharmaceutically acceptable auxiliary substance capable of swelling the pharmaceutical composition to density below 1 g/cm³ at pH between 1.5 and 3.5, at least one pharmaceutically acceptable auxiliary substance capable of ensuring sustained release of the active ingredient, and at least one pharmaceutically acceptable component capable of forming carbon dioxide upon contact with gastric juices.

2. The pharmaceutical composition according to claim 1, wherein the active ingredient effective in stomach is rebamipide.

3. The pharmaceutical composition according to any one of the preceding claims, wherein the pharmaceutically acceptable auxiliary substance capable of swelling the pharmaceutical composition to density below 1 g/cm³ at pH between 1.5 and 3.5 is crospovidone, which is preferably present in the amount of 5 to 15 wt. %, relative to the total weight of the composition.

4. The pharmaceutical composition according to any one of the preceding claims, wherein the auxiliary substances capable of ensuring sustained release of the active ingredient include cellulose and cellulose ethers such as hydroxypropylcellulose, hydroxypropyl methylcellulose, sodium carboxymethylcellulose, ethylcellulose; alginic acid and alginates, such as propylene glycol alginate or sodium alginate; polymethacrylate polymers; guar gum; xanthan gum.

5. The pharmaceutical composition according to any one of the preceding claims, wherein the auxiliary substance capable of ensuring sustained release of the active ingredient is
- an alginate such as propylene glycol alginate, preferably in an amount of between 10 and 50 wt. %, more preferably between 15 and 35 wt. %, relative to the total weight of the composition, and/or
- a cellulose ether, preferably in an amount of between 3 and 15 wt. %, more preferably between 5 and 10 wt. %, relative to the total weight of the composition.

6. The pharmaceutical composition according to any one of the preceding claims, wherein the pharmaceutically acceptable component capable of forming carbon dioxide upon contact with gastric juices is selected from carbonates and hydrogen carbonates of alkali metals and alkaline earth metals, which are preferably present in an amount in the range from 2 to 15 wt. %, more preferably from 5 to 10 wt. %, relative to the total weight of the composition.

7. The pharmaceutical composition according to any one of the preceding claims, in which at least one component is a pharmaceutically acceptable component capable of adhering to the stomach mucose, preferably the mucoadhesive component is the alginate such as propylene glycol alginate.

8. The pharmaceutical composition according to claim 1, which comprises
- at least one active ingredient effective in stomach, preferably rebamipide, in an amount of 20-50 wt. %, more preferably 30-40 wt.%,
- at least one cellulose ether such as hydroxypropylcellulose, hydroxypropyl methylcellulose, ethylcellulose;
- at least one alginate such as propylene glycol alginate, sodium alginate, or alginic acid;
- at least one carbon dioxide-forming component such as sodium hydrogencarbonate, sodium carbonate, calcium hydrogencarbonate, calcium carbonate,
- crospovidone,
- at least one lubricant such as magnesium stearate, calcium stearate, stearic acid, sodium laurylsulphate,
- at least one filler/diluent and/or disintegrant such as saccharide alcohols (mannitol, sorbitol, xylitol), lactose, starch, cellulose, sodium cross-carmelose, sodium carboxymethyl starch.

9. The pharmaceutical composition according to claim 1, which comprises
- at least one active ingredient effective in stomach, preferably rebamipide, in an amount of 20-50 wt. %, more preferably 30-40 wt.%, and the following pharmaceutically acceptable auxiliary substances and components:
- 3-15 wt.% of at least one cellulose ether such as hydroxypropyl methylcellulose, hydroxypropylcellulose, ethylcellulose,
- 10-50 wt.% of at least one alginate such as propylene glycol alginate, sodium alginate, or alginic acid,
- 2-15 wt.% of at least one carbon dioxide-forming component such as sodium hydrogencarbonate, sodium carbonate, calcium hydrogencarbonate, calcium carbonate,
- 5-15 wt.% of crospovidone,
- 0.5-3 wt.% of at least one lubricant such as magnesium stearate, calcium stearate, stearic acid, sodium laurylsulphate,
- 5-20 wt.% of at least one filler/diluent and/or disintegrant such as saccharide alcohols (mannitol, sorbitol, xylitol), lactose, starch, cellulose, sodium cross-carmelose, sodium carboxymethyl starch.

10. The pharmaceutical composition according to claim 1, which is in the form of a tablet, and comprises
- at least one active ingredient effective in stomach, preferably rebamipide, in an amount of 30-40 wt.%,
- 3-15 wt.% of hydroxypropyl methylcellulose and/or hydroxypropylcellulose,
- 10-50 wt.% of propylene glycol alginate, sodium alginate, and/or alginic acid,
- 5-15 wt.% of sodium hydrogencarbonate, sodium carbonate, and/or calcium carbonate,
- 5-15 wt.% of crospovidone,
- 0.5-3 wt.% of magnesium stearate, calcium stearate, and/or stearic acid,
- 5-20 wt.% of mannitol, sorbitol, xylitol, lactose, starch, and/or sodium carboxymethyl starch.

11. The pharmaceutical composition according to claim 1, which is in the form of a tablet, and comprises
- at least one active ingredient effective in stomach, preferably rebamipide, in an amount of 30-40 wt.%,
- 5-10 wt.% of hydroxypropyl methylcellulose,
- 15-35 wt.% of propylene glycol alginate,
- 5-10 wt.% of sodium hydrogencarbonate, sodium carbonate, and/or calcium carbonate,
- 5-15 wt.% of crospovidone,
- 0.5-3 wt.% of magnesium stearate, calcium stearate, and/or stearic acid,
- 5-20 wt.% of mannitol, sorbitol, and/or xylitol.

12. A method of preparation of the pharmaceutical composition of any one of the preceding claims, which comprises the steps of wet granulation and/or dry homogenization of the active ingredient with the auxiliary substances and components.

13. The method according to claim 12, wherein the pharmaceutical composition is prepared by wet granulation of the active ingredient, preferably rebamipide, with a filler/diluent and crospovidone, drying, and homogenization of the thus obtained granules with cellulose ether, alginate, crospovidone, carbon dioxide-forming component and lubricant.

14. The method according to claim 13, wherein the homogenization is carried out in three steps, in the following order: homogenization of the granulate with cellulose ether, homogenization of the thus obtained homogenizate with alginate and carbon-dioxide-forming component, and homogenization of the thus obtained homogenizate with the lubricant.
